# EUROPÄISCHE PATENTSCHRIFT

(11) **EP 0 848 964 B1**
(45) Veröffentlichungstag und Bekanntmachung des Hinweises auf die Patenterteilung: **25.07.2007**
(21) Anmeldenummer: 96120287.6
(22) Anmeldetag: 17.12.1996
(51) Int. Cl.: A61M 39/24, F16K 15/14

(54) **Rückschlagventil, insbesondere für die Medizintechnik**
Check valve especially for medical use
Soupape anti-retour pour utilisation médicale

(43) Veröffentlichungstag der Anmeldung: 24.06.1998
(73) Patentinhaber: Filtertek B.V., County of Limerick (IE)
(72) Erfinder: Myers, Jan Willem Marinus Ing., 5913 TP Venlo (NL)
(74) Vertreter: Brose, D. Karl

(56) Entgegenhaltungen:
- WO-A-96/03166
- DE-A- 4 039 814
- DE-A- 4 142 494
- DE-A- 4 304 949
- DE-A- 4 309 262
- DE-U- 9 319 810
- US-A- 4 222 407
- US-A- 4 286 628
- US-A- 4 369 812
- US-A- 5 025 829

## Beschreibung

Die Erfindung betrifft ein Rückschlagventil nach dem Oberbegriff des Anspruchs 1.

Ein derartiges Rückschlagventil ist aus der US-A-5 025 829 bekannt.

Ausgehend von einem Rückschlagventil der oben genannten Art liegt der Erfindung daher die Aufgabe zu Grunde, dieses bezüglich der Ventilfunktion im angegebenen Druckbereich ausgesprochen sicher zu gestalten, so dass dieses die hohen, in der Medizintechnik gestellten Anforderungen erfüllt, wobei zugleich eine automatische Montage in hohen Stückzahlen möglich ist.

Diese Aufgabe wird erfindungsgemäß im Wesentlichen durch die Merkmale des Kennzeichens des Anspruchs 1 gelöst.

Eine besonders bevorzugte Ausführungsform nach der Erfindung kann dadurch geschaffen werden, dass die Membranscheibe einen Durchmesser von etwa 10 mm und eine Dicke von etwa 0,2 - 0,5 mm aufweist. Hierbei sind Dicke und Durchmesser besonders vorteilhaft aufeinander abgestimmt.

In vorteilhafter Weise bestehen das erste und das zweite Schlauchanschlussgehäuse aus Kunststoff und die Schlauchanschlussgehäuse sind unmittelbar miteinander verschweißt.

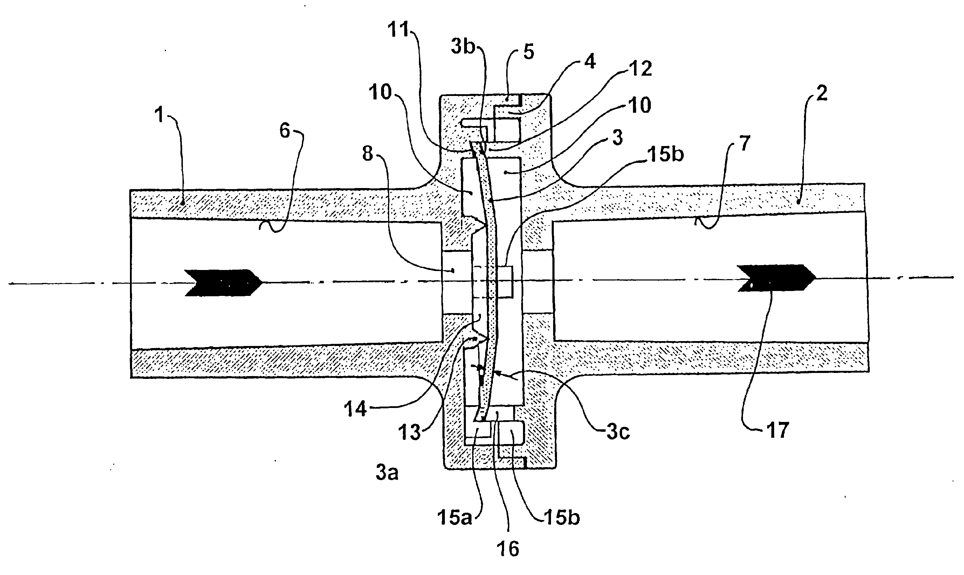

Im Folgenden wird die Erfindung an Hand einer in der Zeichnung beispielhaft veranschaulichten Ausführungsform näher erläutert.

Die einzige Figur der Zeichnung zeigt das Rückschlagventil in einem axialen Längsschnitt.

Das Rückschlagventil weist ein erstes Schlauchanschlussgehäuse 1 und ein zweites Schlauchanachlusagehäuse 2 auf. Zwischen beiden Gehäusen 1 und 2 liegt eine Membranscheibe 3 aus Flüssig-Silikon, Silikon oder Naturgummi. Das Rückschlagventil ist insbesondere für die Medizintechnik geeignet, kann aber auch in anderen Bereichen der Fluidtechnik angewendet werden. Eine Anwendung in der Mikro-Pneumatik ist ebenso vorteilhaft möglich wie in der Mikro-Hydraulik.

Das Schlauchanschlussgehäuse 2 ist mit einem Innenringschaft 4 und das Sclilauchanschlussgehäuse 1 mit einem Außenringschaft 5 versehen. In dem Schlauchanachlussgehäuse 1 ist außerdem ein Eingangsraum 6 und in dem Schlauchanschlussgehäuse 2 ein Ausgangsraum 7 gebildet, die einen größtmöglichen Innendurchmesser gegenüber dem Außendurchmesser aufweisen. Der Eingangsraum 6 vermindert sich in Strömungsrichtung 17 bis auf einen kleineren Durchmesser einer Eingangsöffnung 8, der auf der anderen Seite der Membranscheibe 3 eine Ausgangsöffnung, die mit dem Ausgangsraum 7 verbunden ist, gegenüberliegt.

Die kreisrunde Membranscheibe 3 ist aus Flüssig-Silikon, Silikon oder Naturgummi hergestellt, das bei Normaltemperatur äußerst elastisch ist und nicht klebt (mangels elektrostatischer Aufladung). Die Dicke 3c des Membranquerschnitts 3a ist gleichmäßig mit einer Toleranz gewählt, die sich aus dem Herstellverfahren einer Flüssig-Silikon-, Silikon- oder Naturgummi-Bahn oder einer Flüssig-Silikon-, Silikon - oder Naturgummi-Matte ergibt.

Ein Ventilraum 10 ist durch einen an dem ersten Schlauchanschlußgehäuse 1 angebrachten Membransitzring 11 und durch einen am zweiten Schlauchanschlußgehäuse 2 angebrachten Preßring 12 abgetrennt, wobei die kreisrunde Membranscheibe 3 bei einem Durchmesser von ca. 10 mm eine Dicke 3c des Membranquerschnitts 3a von 0,2-0,5 mm aufweist und eingespannt ist. Dabei sitzt die Membranscheibe 3 mit einem Ringrand 3b zwischen dem Membransitzring 11 des ersten Schlauchanschlußgehäuses 1 und dem Preßring 12 des zweiten Schlauchanschlußgehäuses 2 dichtend. Diese Lage wird durch Verschweißen der beiden Schlauchanschlußgehäuse 1 und 2 fixiert.

Der sich auf beiden Seiten der Membranscheibe 3 befindliche Ventilraum 10 ist ringförmig ausgebildet und durch die Membranscheibe 3 geteilt, solange letztere auf einem am ersten Schlauchanschlußgehäuse 1 angeformten Dichtlippenring 13 dichtend aufliegt. Solange in dem Eingangsraum 6 naturgemäß ein höherer Druck herrscht, hebt die Membranscheibe 3 ab und das Fluid strömt durch die Eingangsöffnung 8 und durch einen Dichtlippenkernraum 14 an der Fläche der Membranscheibe 3 vorbei durch einen Ventilraumdurchlaß 15a aus und durch einen Ventilraumdurchlaß 15b und weiter durch einen Preßringdurchlaß 16 in den anderen Teil des Ventilraums 10 (in der Zeichnung in Strömungsrichtung 17 nach rechts).

Sollte, was in der Medizintechnik nicht erwünscht ist, aber auftritt, der Druck in dem Ausgangsraum 7 höher steigen als im Eingangsraum 6, so preßt dieser

Druck im Sinne des Rückschlagventils die Flüssigkeit so gegen die Membranscheibe 3, daß diese sich gegen den Dichtlippenring 13 legt und jeglichen Durchfluß verhindert. Dieser Vorgang geschieht jedoch auch, wenn der Fluidstrom versiegt, so daß durch eine innere Radialspannung der Membranscheibe 3 im Bereich des Dichtlippenrings 13 gegenüber dem "niedriger" angeordneten Bereich des Membransitzringes 11 dieser die Tendenz innehat, immer zu schließen. Diese Eigenschaften sind jedoch nur mit der aus Flüssig-Silikon, Silikon oder Naturgummi hergestellten und durch Stanzen erzeugten Membranscheibe 3 zu erreichen.

## Patentansprüche

1. Rückschlagventil, insbesondere für die Medizintechnik, im fluidischen Druckbereich von 0,1 - 0,02 bar (0,04 psig) mit einem ersten Schlauchanschlussgehäuse (1) und einem zweiten schlauchanschlussgehäuse (2), und mit einer zwischen beiden Schlauchanschlussgehäusen (1, 2) angeordneten kreisrunden Membranscheibe (3) aus einem flexiblen Werkstoff, die bei Überdruck in einem Eingangsraum (6) von einem Dichtlippenring (13) abhebbar ist, wobei ein Durchflussquerschnitt entsteht und die bei Überdruck in einem Ausgangsraum (7) sicher und in Minimalzeiten auf einen Dichtsitz andrückbar ist, um zu schließen, wobei die Membranscheibe (3) aus Flüssig-Silikon, Silikon oder Naturgummi hergestellt ist, wobei die Membranscheibe (3) mit einem Ringrand (3b) zwischen einem Membransitzring (11) des ersten schlauchanschlussgehäuses (1) und einem Pressring (12) des zweiten Schlauchanschlussgehäuses (2) dichtend eingespannt ist, und wobei auf beiden Seiten der Membranscheibe (3) jeweils ein Ventilraum (10) gebildet ist und der gesamte Mittelbereich der Membranscheibe (3) ohne zusätzliche Abstützung frei über die ventilräume (10) gespannt ist, **dadurch gekennzeichnet, dass** die Membranscheibe (3) ohne Unterbrechungen oder Öffnungen durchgehend ausgebildet ist, dass die Ventilräume (10) jeweils mit einem oder mehreren Ventilraumdurchlässen (15a, 15b) versehen sind, und dass ein oder mehrere über den Umfang verteilte Pressringdurchlässe (16) vorgesehen sind, wobei die Dicke (3c) des Membranquerschnitts (3a) gleichmäßig mit einer Toleranz gewählt ist, die sich aus dem Herstellverfahren einer Flüssig-Silikon-, Silikon- oder Naturgummi-Bahn oder einer Flüssig-Silikon-, Silikon- oder Naturgummi-Matte ergibt..

2. Rückschlagventil nach Anspruch 1, **dadurch gekennzeichnet, dass** die Membranscheibe (3) einen Durchmesser von etwa 10 mm und eine Dicke (3c) von etwa 0,2 - 0,5 mm aufweist.

3. Rückschlagventil nach Anspruch 1 oder 2, **dadurch gekennzeichnet, dass** das erste und das zweite Schlauchanschlussgehäuse (1, 2) aus Kunststoff bestehen, und dass die Schlauchanschlussgehäuse (1, 2) unmittelbar miteinander verschweißt sind.

## Claims

1. Check valve, especially for the medical technique, in the fluid pressure range of 0,1 - 0,02 bar (0,04 psig) having a first hose connector housing (1) and a second hose connector housing (2) and a circular diaphragm disk (3) of a flexible material being positioned between the two hose connector housings (1, 2) which, at an over-pressure, in an entry space (6) can be lifted from a sealing lip ring (13), wherein a flow cross-section is formed and which, at an over-pressure, in an exit space (7) safely and in minimal times can be pressed on a sealing seat to close the valve, wherein the diaphragm disk (3) is made from liquid silicon, silicon or natural rubber, wherein the diaphragm disk (3) with an annular rim (3b) sealingly is clamped between an annular diaphragm seat (11) of the first hose connector housing (1) and a pressure ring (12) of the second hose connector housing (2) and, wherein on both sides of the diaphragm disk (3) each a valve space (10) is formed and the entire central area of the diaphragm disk (3) is freely tensioned over the valve spaces (10) without any additional support, **characterised in that** the diaphragm disk (3) is formed without any interruptions or openings in a continuous way, that the valve spaces (10) each are provided with one or more valve space passages (15a, 15b) and, that one or more passages (16) in the pressure ring are provided distributed over the circumference thereof, wherein the thickness (3c) of the diaphragm disk cross-section (3a) uniformly is chosen with a tolerance resulting from the manufacturing method of a liquid silicon-, silicon- or natural rubber-sheet or a liquid-silicon-, silicon- or natural rubber-mat.

2. Check valve according to claim 1, **characterised in that** the diaphragm disk (3) is having a diameter of about 10 mm and a thickness (3c) of about 0,2 - 0,5 mm.

3. Check valve according to claim 1 or 2, **characterised in that** the first and the second hose connector housing (1, 2) are consisting of plastics and, that the hose connector housings (1, 2) directly are welded together.

## Revendications

1. Clapet antiretour, en particulier pour la technique médicale, dans la plage de pression fluidique de 0,1 à 0,02 bar (0,04 psig) comprenant un premier boîtier de raccordement de flexible (1) et un second boîtier de raccordement de flexible (2), et avec un disque membrane (3) circulaire, disposé entre les deux boîtiers de raccordement de flexible (1, 2), en un matériau flexible, qui peut être soulevé d'une lèvre d'étanchéité annulaire (13) en cas de surpression dans un espace d'entrée (6), une section d'écoulement se formant, et qui, en cas de surpression dans un espace de sortie (7), peut être appuyé dans des espaces de temps minimum sur un siège étanche afin de fermer, le disque membrane (3) étant fabriqué à base de silicone liquide, de silicone ou de caoutchouc naturel, le disque membrane (3) étant fixé de façon étanche avec un bord annulaire (3b) entre un siège annulaire de membrane (11) du premier boîtier de raccordement de flexible (1) et un anneau de serrage (12) du second boîtier de raccordement de flexible (2), et une chambre de clapet (10) étant formée sur chacun des deux côtés du disque membrane (3) et l'ensemble de la zone centrale du disque membrane (3) étant tendu sans soutien supplémentaire librement sur les chambres de clapet (10), **caractérisée en ce que** le disque membrane (3) est conçu de façon continue sans interruptions ou ouvertures, **en ce que** les chambres de clapet (10) sont dotées chacune d'un ou de plusieurs passages de chambre de clapet (15a, 15b) et **en ce qu'**un ou plusieurs passages d'anneau de serrage (16) répartis sur le pourtour sont prévus, l'épaisseur (3c) de la section de membrane (3) étant choisie uniformément avec une tolérance qui est obtenue à partir du procédé de fabrication d'une bande en silicone liquide, en silicone ou en caoutchouc naturel ou d'un tapis en silicone liquide, en silicone ou en caoutchouc naturel.

2. Clapet antiretour selon la revendication 1, **caractérisée en ce que** le disque membrane (3) présente un diamètre d'environ 10 mm et une épaisseur (3c) d'environ 0,2 à 0,5 mm.

3. Clapet antiretour selon la revendication 1 ou 2, **caractérisée en ce que** le premier et le second boîtiers de raccordement de flexible (1, 2) sont en matière plastique et **en ce que** les boîtiers de raccordement de flexible (1, 2) sont soudés directement entre eux.
